Europäisches Patentamt

European Patent Office

Office européen des brevets

(11) Publication number: **0 524 796 B1**

## EUROPEAN PATENT SPECIFICATION

(45) Date of publication of patent specification: **12.04.95**

(51) Int. Cl.6: **A61K 31/20**

(21) Application number: **92306646.8**

(22) Date of filing: **20.07.92**

(54) **Use of essential fatty acids in the preparation of a medicament for the treatment of AIDS.**

(30) Priority: **24.07.91 GB 9116054**

(43) Date of publication of application:
**27.01.93 Bulletin  93/04**

(45) Publication of the grant of the patent:
**12.04.95 Bulletin  95/15**

(84) Designated Contracting States:
**AT BE CH DE DK ES FR GB GR IT LI LU MC NL PT SE**

(56) References cited:
**DE-A- 4 017 979**

**MEDICAL HYPOTHESES vol. 20, no. 1, 1986, pages 1 - 8; M.E. BEGIN ET AL.: 'Adeficiency in dietary gamma-linolenic and/or eicosapentaenoic acid maydetermine individual susceptility to AIDS'**

**PROSTAGLANDINS LEUKOTRIENS AND ESSENTIAL FATTY ACIDS vol. 37, no. 2, August1989, pages 135 - 137; M.E. BEGIN ET AL.: 'Plasma fatty acid levels in patientswith AIDS and in controls'**

(73) Proprietor: **SCOTIA HOLDINGS PLC
Efamol House
Woodbridge Meadows
Guildford
Surrey GU1 1BA (GB)**

(72) Inventor: **Horrobin, David Frederick
c/o Efamol House,
Woodbridge Meadows
Guildford,
Surrey GU1 1BA (GB)**
Inventor: **Winther, Michael David, c/o Efamol
Research Inc.
Annapolis Valley Industrial Park,
PO Box 818
Kentville,
Nova Scotia B4N 4H8 (CA)**

(74) Representative: **Sturt, Clifford Mark et al
J. MILLER & CO.
34 Bedford Row
Holborn
London WC1R 4JH (GB)**

Rank Xerox (UK) Business Services
(3.10/3.09/3.3.3)

TUMOUR BIOLOGY vol. 9, 1988, pages 225 - 232; E.A. NUNEZ: 'Modulation of cell-mediated immune response by steroids and free fatty acids in AIDS patients: A critical survey'

VOX SANG. vol. 54, 1988, pages 14 - 20; B. HOROWITZ ET AL.: 'Inactivation oflipid-enveloped viruses in labile blood derivatives by unsaturated fatty acids'

J. NUTR. vol. 122, no. 3SUP, March 1992, pages 716 - 722; G. FERNANDES ET AL.:'Potential of Diet Therapy on Murine AIDS'

Annales de Medecine Interne, vol. 142(4), 307-308, 19. July 1991

**Description**

**FIELD OF INVENTION**

The invention relates to the preparation of fatty acid medicaments.

**FATTY ACIDS**

The pathways of conversion of the main series of polyunsaturated fatty acids in the body are as in Table 1 below:

## TABLE 1

| n-6 | | n-3 |
|---|---|---|
| 18:2 delta-9,12 | | 18:3 delta-9,12,15 |
| (linoleic acid) | | (alpha-linolenic acid) |
| | delta-6 desaturase | |
| 18:3 delta-6,9,12 | | 18:4 delta-6,9,12,15 |
| (gamma-linolenic acid) | | (stearidonic acid) |
| | elongation | |
| 20:3 delta-8,11,14 | | 20:4 delta-8,11,14,17 |
| (dihomo-gamma-linolenic acid) | | |
| | delta-5 desaturase | |
| 20:4 delta-5,8,11,14 | | 20:5 delta-5,8,11,14,17 |
| (arachidonic acid) | | ('eicosapentaenoic acid') |
| | elongation | |
| 22:4 delta-7,10,13,16 | | 22:5 delta-7,10,13,16,19 |
| (adrenic acid) | | |
| | delta-4 desaturase | |
| 22:5 delta-4,7,10,13,16 | | 22:6 delta-4,7,10,13,16,19 |
| | | ('docosahexaenoic acid') |

3

The above pathways are not normally reversible nor, in man, are n-3 and n-6 series acids inter-convertible.

The acids, which in nature are of the all-cis configuration, are systematically named as derivatives of the corresponding octadecanoic, eicosanoic or docosanoic acids, e.g. delta-9,12-octadecadienoic acid or delta-4,7,10,13, 16,19-docosahexaenoic acid, but numerical designations such as, correspondingly, 18:2 n-6 or 22:6 n-3 are convenient. Initials, for example, EPA for the 20:5 n-3 acid (eicosapentaenoic acid) or DHA for the 22:6 n-3 acid (docosahexaenoic acid), are also used but do not serve when n-3 and n-6 acids of the same chain length and degree of unsaturation exist as for example with the 22:5 acids. Trivial names in more or less common use in the n-6 series are as shown. Of the n-3 series only 18:3 n-3 has a commonly used trivial name, alpha-linolenic acid, though the name stearidonic acid is coming into use for the 18:4 n-3 acid and the names eicosapentaenoic acid and docosahexanenoic acid as such are also used. The alpha isomer of linolenic acid was characterised earlier than gamma-linolenic acid and reference in the literature simply to linolenic acid, especially in the earlier literature, is to the alpha-acid.

## GENERAL DISCUSSION OF INVENTION

Acquired Immune Deficiency Syndrome (AIDS) is believed to be caused by a virus, Human Immuno-deficiency Virus (HIV), which has many different strains and which is particularly damaging to the immune system. This damage to the immune system makes it difficult for the AIDS sufferer to resist infections of many different types. The damage to the immune system is frequently monitored by measuring the levels of a particular class of lymphocyte, the CD4 lymphocyte, in the blood.

AIDS causes directly and indirectly severe and extensive damage to many body systems. The role that damage to each system plays in the development of the many and various symptoms of AIDS has not been clearly defined. Very large numbers of biochemical and pathological abnormalities have been described, but it is likely that most of these are consequences of the disease process and are not causally involved in the development of symptoms.

It has been suggested that EFA deficiencies may be important in AIDS (M.E. Begin, U.N Das, Med. Hypotheses 20: 1-8, 1986)). Further, we have reported that in AIDS patients the levels of essential fatty acids (EFAs) in the total lipid and phospholipid fractions of the blood are unusually low (M.E. Begin, M.S. Manku and D.F. Horrobin, "Prostaglandins & Leukotrienes" 37 135-137 (1989)). EFAs are thought to be important to many of the physiological and pathological reactions of the body. It seemed unlikely to us that the EFA deficits could explain many of the symptoms of AIDS but we felt that the possibility could not be ruled out and so developed the hypothesis that supplementation with appropriate EFAs might provide relief of symptoms or prevent progression of the disease in patients with AIDS or in patients infected with the HIV virus who had not yet developed AIDS.

There are two series of EFAs, the n-6 derived from linoleic acid, and the n-3 derived from alpha-linolenic acid. However, the parent EFAs have only limited roles in the body. Most of their effects depend on the metabolism of linoleic and alpha-linolenic acids to further metabolites by the delta-6-desaturase enzyme. Delta-6-desaturation is severely rate limiting in humans. Since the biochemical studies showed that the patients with AIDS were deficient in the metabolites of the EFAs which had undergone 6-desaturation, we decided to by-pass this rate limiting step by administering gamma-linolenic acid (GLA) or higher acids of the n-6 series and stearidonic acid or higher acids such as eicosapentaenoic acid (EPA) of the n-3 series.

GLA was given in the form of evening primrose oil in a formulation which provided 32 mg of GLA per capsule. EPA was given in the form of fish oil which provided 16 mg of EPA and 10 mg of its further metabolite, docosahexaenoic acid, per capsule. The GLA and the EPA were incorporated into the same capsules. Twelve capsules per day were administered, giving a total daily dose of around 388 mg of GLA, 192 mg of EPA and 120 mg of DHA. In addition each capsule contained 350 mg of linoleic acid, making a total dose of 4200 mg of linoleic acid per day. Thus the total dose of n-6 EFAs was 4588 mg/day, 388 mg in the form of GLA. The total dose of n-3 fatty acids was 312 mg/day. Since the n-6 EFAs are more important than the n-3 EFAs, and since the n-3 EFAs can compete with the n-6 EFAs at certain important metabolic steps, it is preferable to have more n-6 EFAs than n-3 EFAs in a formulation and it is especially preferable to have more n-6 EFAs which have undergone 6-desaturation than n-3 EFAs which have undergone 6-desaturation.

Twelve patients attending the AIDS Clinic at Muhimbili Hospital, the University Hospital of Dar-es-Salaam, Tanzania, were entered into a study. They all had severe symptomatic AIDS, with weight loss, fatigue and diarrhoea being the predominant symptoms. They scored the severity of fatigue and diarrhoea on a simple four point scale (0 = normal, 1 = mild, 2 = moderate, and 3 = severe). Their scores were recorded at entry to the study and after eight and twelve weeks. At the beginning of the study and again after eight weeks blood was taken for assay of CD4 lymphocytes.

The results are shown in Table 2 and demonstrate a clear benefit for the treatment with EFAs. The patients on average gained weight and experienced substantial reduction in fatigue and diarrhoea. At the end of twelve weeks the doctor, in consultation with the patients, rated the patient in relation to the condition at baseline as worse, , unchanged, better or much better. Two patients became worse, three remained unchanged, five were rated as better and two were much better, being so improved that they could actually return to work. At the same time the mean CD4 count in the group rose significantly ($p < 0.05$. Student's $\underline{t}$ test), providing objective support for the subjective evidence of improvement.

## Table 2

Severity of symptoms and CD4 lymphocyte levels in twelve patients with AIDS before and during treatment with GLA and EPA. Symptom scores represent the mean ± SEM for twelve patients as do the lymphocyte counts.

| Parameter | Baseline | Week 8 | Week 12 |
|---|---|---|---|
| Weight (kg) | 62.1 ± 2.9 | 62.0 ± 3.5 | 64.4 ± 6.2 |
| Fatigue | 0.82 ± 0.16 | 0.40 ± 0.16 | 0.29 ± 0.18 |
| Diarrhoea | 0.50 ± 0.15 | 0.20 ± 0.13 | 0.14 ± 0.14 |
| CD4 lymphocytes (cells/ μl) | 59 ± 12.5 | 261 ± 63.7 | Not done |

### STATEMENT OF INVENTION

The invention lies in a combined use of essential fatty acids of the n-6 series and of the n-3 series in the preparation of a medicament for relieving the symptoms of AIDS, or of preventing the development of symptoms in HIV infected persons, or of raising the CD4 lymphocyte count or preventing its deterioration. As essential fatty acids (EFAs) are used, especially EFAs which have undergone 6-desaturation, such as gamma-linolenic acid (GLA), dihomo-gamma-linolenic acid (DGLA) and arachidonic acid (AA) of the n-6 group, and stearidonic acid (SA), eicosapentaenoic acid (EPA), docosapentaenoic acid (DPA) and docosahexaenoic acid (DHA) of the n-3 group, all of which are present only at low levels in the blood of AIDS patients.

In particular we provide the use of GLA and EPA.

It is preferred that the ratio of 6-desaturated n-6 to 6-desaturated n-3 EFAs should desirably not be less than 1:1 and should preferably be in the range of from 1.2:1 to 6:1.

### DOSE RANGE

1 mg to 100 g each of the said fatty acids, but especially of GLA or EPA, preferably 50 mg to 10 g, and very preferably 200 mg to 2 g per day, desirably within the above ratios.

### DERIVATIVES OF EFAs

The acids may be used as such or as pharmaceutically acceptable and physiologically equivalent derivatives, and reference to any of the acids is to be taken as including reference to the acids when in the form of such derivatives. Thus EFAs may be provided as free fatty acids, mono-, di- or triglycerides, salts including lithium salts; amides; esters; phospholipids; or any other physiologically acceptable form which can be demonstrated to enter the body by its ability to raise within the blood or other body tissue the level of the EFA and/or of its metabolites.

Specifically, equivalence is demonstrated by entry into the pathway quoted herein, as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al, page 23, "Analysis of Lipids and Lipoproteins" Ed Perkins, American Oil Chemists Society, Champaign, Illinois, U.S.A.

In outline the method is suitably that plasma samples (1 ml) are extracted with chloroform:methanol (2:1). The extract is filtered through sodium sulphate, evaporated to dryness, and taken up in 0.5 ml chloroform:methanol. The lipid fractions are separated by thin layer chromatography or silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot column packed with 10% silar on chromosorb WAW 106/230. The carrier gas is helium (30 ml/min). Oven temperature is programmed to rise from 165°C to 190°C at 2°C/min. Detector temperature is 220°C and injector temperature 200°C. Retention times and peak areas are automatically computed by Hewlett-Packard Level 4 integrator. Peaks are identified by comparison with standard fatty acid methyl esters.

## DIETARY COMPOSITIONS

The invention is chiefly described in terms of methods of treatment and pharmaceutical compositions, but it will be understood that the gamma-linolenic and other EFAs, being in the nature of dietary supplements, can be incorporated in a dietary margarine or other foodstuff and such are to be understood as within the term pharmaceutical composition or medicament herein (including in the claims) when for the purposes set out.

## FORMS AND SOURCES OF GAMMA-LINOLENIC AND OTHER ACIDS

If desired, pharmaceutical compositions may be produced for use in the invention by associating the natural or synthetic acids, as such or as derivatives, with an acceptable pharmaceutical vehicle. It is, however, at present convenient to provide at least GLA in the form of an available oil having a high GLA content, hence reference to "oils" herein.

One source of oils currently available is the seed of evening primrose species such as Oenothera biennis L. and Oenothera lamarckiana, the oil extract therefrom containing about 8% GLA and about 72% linoleic acid in the form of their glycerides, together with other glycerides (percentages based on total fatty acids). Other sources of GLA are borage species such as Borago officinalis which, though current yield per acre is low, provide a richer source than Oenothera oil. Oils from the seeds of members of the Ribes family are also often rich in GLA. Recent studies on fungi which can be cultivated by fermentation promise a fungal oil source.

The oil is extracted from the seed by one of the conventional methods of extraction such as cold pressure, screw pressure after partially cooking the seed, or solvent extraction.

Fractionation of a typical sample of this oil in the form of methyl esters shows the relative proportions:

| | |
|---|---|
| Palmitate | 6.15 |
| Stearate | 1.6 |
| Oleate | 10.15 |
| Linoleate | 72.6 |
| Gamma-linolenate | 8.9 |

The seed oil extracts referred to above can be used as such or can, for example, if desired, be fractionated to yield an oily composition containing the triglycerides of gamma-linolenic and linoleic acids as the main fatty acid components, the gamma-linolenic acid content being, if desired, a major proportion. Seed oil extracts appear to have a stabilising effect upon DGLA if present.

## SOURCES OF OTHER ACIDS

DGLA can be prepared by chemical synthesis or by fungal fermentation. For the higher n-6 acids, natural sources of 22:4 and 22:5 n-6 acids include adrenal glands (22:5) and kidneys (22:4) obtained from slaughter houses, which also give AA sources.

The n-3 acids are available from natural plant sources in for example the case of alpha-linolenic acid; from marine oils, particularly the 20:5 n-3 and 22:6 n-3 acids, and more recently from microbial fermentation. They can be isolated from these sources by, for example, saponification under mild non-oxidising conditions followed by preparative gas liquid chromatography. Synthesis is difficult but not impossible and provides another source.

**ROUTE OF ADMINISTRATION**

Any of the accepted topical, oral, enteral, parenteral, rectal, vaginal or other route suitable for pharmaceutical use. Such compositions, and for any particular kind of preparation, are very well known. Thus, for example, tablets, capsules, ingestible liquid or powder preparations can be prepared as required, and topical preparations also when the gamma-linolenic acid or other acids are to be absorbed through the skin. Injectable solutions of hydrolysed Oenothera oil may be prepared using albumin to solubilise the free acid, or injectable emulsions can be made by using appropriate phospholipids and other emulsifiers known to those skilled in the art.

Advantageously, a preservative is incorporated into the preparation. Alpha-tocopherol in concentration of about 0.1% by weight has been found suitable for the purpose and is one of a number of possible stabilisers well known in the field and including also for example ascorbyl palmitate and stearate.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

**EXAMPLES**

The following are used for treating the symptoms of AIDS, or preventing deterioration, or preventing the development of symptoms in HIV infected persons:-

1. Twelve capsules of a mixture of evening primrose oil and fish oil, providing 32 mg of GLA and 16 mg of EPA.
2. Eight capsules per day, each containing 50 mg GLA, 50 mg AA, 50 mg EPA and 50 mg DHA.
3. A cream, lotion or other formulation for topical administration containing 2% by weight of GLA and 1% by weight of EPA.

**Claims**

1. Use of one or more essential fatty acids of the n-6 series and one or more essential fatty acids of the n-3 series in the preparation of a medicament for relieving the symptoms of AIDS or for preventing the progression of AIDS in HIV infected persons.

2. Use according to claim 1 wherein said acid is selected from acids which have undergone 6-desaturation, particularly from GLA (the preferred n-6 acid), DGLA and AA in the n-6 series and from SA, EPA (the preferred n-3 acid), DPA, DHA in the n-3 series.

3. Use according to claim 1 or 2 wherein GLA and EPA are selected.

4. Use according to any preceding claim, wherein the ratio of n-6 series EFAs to n-3 series EFAs is not less than 1:1 and is desirably in the range of from 1.2:1 to 6:1.

5. Use according to any preceding claim, wherein the medicament is in unit dosage form, suited to the administration of from 1mg to 100 g per day of the or each essential fatty acids, preferably from 50 mg to 10 g, more preferably from 200 mg to 2 g.

6. Use of one or more essential fatty acids of the n-6 series and one or more essential fatty acids of the n-3 series in the preparation of a medicament for raising the CD4 lymphocyte count or for preventing deterioration of the CD4 lymphocyte count in HIV infected persons.

**7.** Use according to claim 6 wherein said acid is selected from acids which have undergone 6-desaturation, particularly from GLA (the preferred n-6 acid), DGLA and AA in the n-6 series and from SA, EPA (the preferred n-3 acid), DPA, DHA in the n-3 series.

**8.** Use according to claim 6 or 7 wherein GLA and EPA are selected.

**9.** Use according to any of claims 6 to 8 wherein the ratio of n-6 series EFAs to n-3 series EFAs is not less than 1:1 and is desirably in the range of from 1.2:1 to 6:1.

**10.** Use according to any of claims 6 to 9, wherein the medicament is in unit dosage form, suited to the administration of from 1mg to 100g per day of the or each essential fatty acids, preferably from 50 mg to 10 g, more preferably from 200 mg to 2 g.

**Patentansprüche**

**1.** Verwendung einer oder mehrerer essentieller Fettsäuren der n-6 Reihen und einer oder mehrerer essentieiler Fettsäuren der n-3 Reihen für die Herstellung eines Medikamentes, um die Symptome von AIDS zu mildern oder um den Fortschritt von AIDS bei HIV infizierten Personen zu verhindern.

**2.** Verwendung gemäß Patentanspruch 1, wobei genannte Säure aus Säuren ausgewählt wird, die 6-desaturiert wurden, insbesondere aus GLS (der bevorzugten n-6 Säure), DGLS und AS in den n-6 Reihen und aus SS, EPS (der bevorzugten n-3 Säure), DPS, DHS in den n-3 Reihen.

**3.** Verwendung gemäß Patentanspruch 1 oder 2, wobei GLS und EPS ausgewählt werden.

**4.** Verwendung gemäß irgendeinem vorangegangenen Patentanspruch, wobei das Verhältnis von EFSs der n-6 Reihen zu EFSs der n-3 Reihen nicht weniger als 1:1 beträgt und wünschenswerterweise in dem Bereich von 1,2:1 bis 6:1 liegt.

**5.** Verwendung gemäß irgendeinem vorangegangenen Patentanspruch, wobei das Medikament in dosierbaren Einheiten vorliegt, die passend für die Verabreichung von 1 mg bis 100 g pro Tag von der oder jeder essentiellen Fettsäure sind, vorzugsweise von 50 mg bis 10 g, stärker bevorzugt von 200 mg bis 2 g.

**6.** Verwendung von einer oder mehreren essentiellen Fettsäuren der n-6 Reihen und einer oder mehreren essentiellen Fettsäuren der n-3 Reihen für die Herstellung eines Medikaments, um die CD4-Lymphozytenanzahl zu steigern oder um eine Verschlechterung der CD4-Lymphozytenanzahl in HIV infizierten Personen zu verhindern.

**7.** Verwendung gemäß Patentanspruch 6, wobei besagte Säure aus Säuren ausgewählt wird, die 6-desaturiert wurden, insbesondere aus GLS (der bevorzugten n-6 Säure), DGLS und AS in den n-6 Reihen und aus SS, EPS (der bevorzugten n-3 Säure), DPS, DHS in den n-3 Reihen.

**8.** Verwendung gemäß Patentanspruch 6 oder 7, wobei GLS und EPS ausgewählt werden.

**9.** Verwendung gemäß irgendeinem der Patentansprüche 6 bis 8, wobei das Verhältnis von EFSs der n-6 Reihen zu EFSs der n-3 Reihen nicht weniger als 1:1 beträgt und wünschenswerterweise im Bereich von 1,2:1 bis 6:1 liegt.

**10.** Verwendung gemäß irgendeinem der Patentansprüche 6 bis 9, wobei das Medikament in dosierbaren Einheiten vorliegt, die passend für die Verabreichung von 1 mg bis 100 g pro Tag von der oder jeder essentiellen Fettsäure sind, vorzugsweise von 50 mg bis 10 g, stärker bevorzugt von 200 mg bis 2 g.

**Revendications**

**1.** Utilisation d'un ou plusieurs acides gras essentiels de la série n-6 et d'un ou plusieurs acides gras essentiels de la série n-3 dans la préparation d'un médicament destiné à soulager les symptômes du SIDA ou à prévenir la progression du SIDA chez les personnes infectées par le HIV.

**2.** Utilisation selon la revendication 1, dans laquelle l'acide est choisi parmi les acides ayant subi une 6-désaturation, notamment parmi le GLA (l'acide en n-6 préféré), le DGLA et l'AA pour la série n-6, et parmi le Sa, l'EPA (l'acide préféré en n-3), le DPA et le DHA pour la série n-3.

**3.** Utilisation selon la revendication 1 ou 2, dans laquelle on choisit le GLA et l'EPA.

**4.** Utilisation selon l'une quelconque des revendications précédentes, dans laquelle le rapport des acides gras essentiels de la série n-6 aux acides gras essentiels de la série n-3 n'est pas inférieur à 1:1 et est si possible compris dans l'intervalle de 1,2:1 à 6:1.

**5.** Utilisation selon l'une quelconque des revendications précédentes, dans laquelle le médicament se présente sous une forme posologique unitaire, convenant à l'administration de 1 mg à 100 g par jour du ou de chacun des acides gras essentiels, de préférence de 50 mg à 10 g, et tout spécialement de 200 mg à 2 g.

**6.** Utilisation d'un ou plusieurs acides gras essentiels de la série n-6 et d'un ou plusieurs acides gras essentiels de la série n-3 lors de la préparation d'un médicament destiné à élever le taux de lymphocytes CD4, ou à prévenir une détérioration du taux des lymphocytes CD4 chez des personnes infectées par le HIV.

**7.** Utilisation selon la revendication 6, dans laquelle l'acide est choisi parmi les acides ayant subi une 6-désaturation, notamment parmi le GLA (l'acide en n-6 préféré), le DGLA et l'AA pour la série n-6, et parmi le SA, l'EPA (l'acide préféré en n-3), le DPA et le DHA pour la série n-3.

**8.** Utilisation selon la revendication 6 ou 7, dans laquelle on choisit le GLA et l'EPA.

**9.** Utilisation selon l'une quelconque des revendications 6 à 8, dans laquelle le rapport des acides gras essentiels de la série n-6 aux acides gras essentiels de la série n-3 n'est pas inférieur à 1:1 et est si possible compris dans l'intervalle de 1,2:1 à 6:1.

**10.** Utilisation selon l'une quelconque des revendications 6 à 9, dans laquelle le médicament se présente sous une forme posologique unitaire, convenant à l'administration de 1 mg à 100 g par jour du ou de chacun des acides gras essentiels, de préférence de 50 mg à 10 g, et tout spécialement de 200 mg à 2 g.